# EUROPEAN PATENT APPLICATION

(11) **EP 2 052 721 A1**
(43) Date of publication of application: **29.04.2009**
(21) Application number: 07020611.5
(22) Date of filing: 22.10.2007
(51) Int. Cl.: A61K 31/337, A61P 19/02

(54) **Use of carnosol for cartilage repair**

(71) Applicant: DSM IP Assets B.V., 6411 TE Heerlen (NL)
(72) Inventor: The designation of the inventor has not yet been filed
(74) Representative: Pressner, Dietmar

(57) **Abstract**

The invention relates to the use of carnosol or a carnosol derivative for inducing or enhancing cartilage repair or cartilage regeneration. Furthermore, the invention relates to nutraceutical and pharmaceutical compositions comprising carnosol or a carnosol derivative for regeneration and repair of cartilage injuries in joints, in particular of traumatic cartilage injuries.

## Description

The invention relates to the use of carnosol or a carnosol derivative for inducing or enhancing cartilage repair or cartilage regeneration. Furthermore the invention relates to nutraceutical and pharmaceutical compositions comprising carnosol or a carnosol derivative for regeneration and repair of cartilage injuries in joints, in particular of traumatic cartilage injuries.

The joint is a point of articulation between two or more bones (*e.g*. knee, ankle, foot, hip, wrist, elbow, shoulder, spinal joints, hand and finger) in an animal including human.

Cartilage is a specialized type of dense connective tissue and is present at several sites in the body for example ears, respiratory tract, intervertebral discs and lining of the end surfaces of bones in joints. Cartilage is an avascular tissue that consists of cells (chondroblasts, chondrocytes), which are embedded in an extensive extracellular matrix (ECM) primarily composed of collagen (e.g., collagen types II, IX and XI), proteoglycans (*e.g*. proteoglycans complexed with chondroitin sulfate, keratan sulfate, hyaluronic acid and dermatan sulfate) and other proteins. There are three main types of cartilage, (i) hyaline cartilage or articular cartilage, (ii) fibrocartilage, (iii) elastic cartilage. These three types differ in structure and function.

Chondrocytes, the only cell type in mature articular cartilage, are terminally differentiated cells that maintain the cartilage-specific matrix phenotype. Under normal conditions they have a low turnover, form collagen types II, IX and XI which are unique to the articular tissue and interact with specific proteoglycans to form the hydrated ECM of hyaline cartilage. For reasons mentioned below, different factors set off imbalances in cartilage physiology. This might affect the cell population that is producing the ECM; alternatively, the homeostasis is disturbed at the level of ECM and leads to an augmented erosion of the ECM due to an insufficient synthesis of ECM components (such as aggrecan, collagen or hyaluronic acid) or - more frequently - to an increased degradation of the ECM.

During early phases of development (fetal and juvenile life) chondrogenesis eventually leads to skeletal development, chondrocyte differentiation and maturation that results in the formation of cartilage and bone during endochondral ossification. This process is controlled by interactions between cells and the surrounding matrix as well as a plethora of growth and differentiation factors which are formed in a tightly regulated temporal and spatial manner. The earliest cartilage differentiates from mesenchymal progenitor cells. These gradually transform into proliferating chondrocytes, become hypertrophic and secrete components of the ECM (aggrecan, hyaluronan, collagens). During bone formation, chondrocytes are transformed into osteoblasts at the perichondrium. Growth factors including *e,g*. FGFs, IGF-1, TGF-beta, BMPs, as well as signaling and transcription factors (SMADs, SOXs, STAT) are sequentially activated during chondrogenesis and subsequent bone formation. While these are the prerequisite for cell lineage specific differentiation events, they are also required for the expression of ECM proteins- For instance, SOX regulates the expression of collagen II and aggrecan and GADD45 regulates apoptosis and terminal differentiation of chondrocytes and might counter-balance the effects of MMP-9.
(i) At the cessation of bone growth, hyaline or articular cartilage covers the epiphyses, *i.e*. the articular surfaces of bones in joints. It plays an essential role in the movement of mammalian joints by enabling bones to move smoothly over one another. Hyaline cartilage consists of chondrocytes lying in an abundant ECM that is composed primarily of water, type II collagen and negatively charged proteoglycans forming an organized structure based on the orientation of the collagen fibers. Thus, the hyaline cartilage acts as a sort of shock-absorbing structure which can withstand compression, tension and shearing forces, and dissipate load and also provides an almost friction-free articulating surface.
(ii) Fibrocartilage includes interarticular fibrocartilage, fibrocartilaginous disc (e.g. meniscus), connecting fibrocartilage and circumferential fibrocartilage. In fibrocartilage, the micro-polysaccharide network is interlaced with prominent collagen bundles, and the chondrocytes are more widely scattered than in hyaline or articular cartilage. Interarticular fibrocartilage is found in joints which are exposed to concussion and subject to frequent movement such as the meniscus of the knee. The meniscus plays a crucial role in joint knee stability, lubrification and force transmission. Fibrocartilaginous discs, which adhere closely to the epiphyses, are composed of concentric rings of fibrous tissue, with cartilaginous laminae interposed (e.g. the intervertebral disc of the spine).
(iii) Elastic cartilage contains fibers of collagen that are histologically similar to elastin fibers. It is primarily found in the epiglottis, the external ear, and the auditory tube.

Cartilage maintenance requires a healthy turnover of tissue components, which needs a delicate balance between anabolic (build-up) and catabolic (break down) events, in order to prevent hypertrophy or excessive degradation of extracellular matrix (ECM), respectively. The ECM is built up of collagen and proteoglycans that are the products of collagen genes or aggrecan genes which are active during anabolic events. Also, chondrocyte proliferation and renewal can be favored by the expression of SOX transcription factors including SOX-5, SOX-6 and SOX-9.

Matrix metalloproteinases (MMPs) are instrumental for organ and tissue remodeling. The tissue-specific expression of the different MMP family members confer them a large variety of roles. While the degradation of ECM is associated with expression of MMP3 or MMP13, other MMPs activate latent signalling molecules (like VEGF, IGF) and trigger changes in tissue architecture or promote cell-migration. A balanced ECM turnover is a prerequisite for maintaining cartilage health. A net ECM degradation due to excess MMP activity can lead to tissue destruction, degeneration and loss of function.

Cartilage injuries are mainly due to trauma and can occur in any joint. They can result from an acute traumatic injury (usually as a result of shear forces) especially in high-impact and/or pivoting sports and are often associated with ligament rupture, damage or disuse. Cartilage injuries are also the result of repetitive excessive mechanical stress to the joint as is the case during intense physical training programs of athletes and military members. Subjects exercising sports with rotational, rapid directional change and loading stresses (*e.g*. runners, football players, weight lifters, tennis players, basketball, handball, ski practice and shooters) are at increased risk of cartilage injuries. The knee and the ankle joints are very frequently injured in athletes mainly following an acute trauma and can result in chronic disability of the athletes if left untreated. Younger, skeletally immature athletes are even at increased risk of cartilage injuries. Moreover, younger adults are also more likely to participate in vigorous physical activity and thus are more likely to be injured. Thus, cartilage injuries are frequent causes of disability in subjects with a long practice of high impact sports. The cartilage injury severity is dependent on the peak stress, strain and strain rate of tissue compression, duration of injurious mechanical loading, and to the biochemical properties and status of maturation of the cartilage tissue. This is presumably further dependent on the genetic constitution of individuals.

Mechanical cartilage injury results in fissure or fibrillation of the articular surface and disruption of the collagen network. As a consequence, proteoglycans are more hydrated; this leads to cartilage thickening and softening. This results in a decreased capacity to absorb shock and support weight load, which further disrupts collagen framework and aggravates the cartilage defects. These appear as a gap, cavity, hole or other substantial disruption in the structural integrity of the cartilage tissue. It can also be a detachment of the cartilage from its point of adhesion to the bone or ligaments. Mechanical cartilage injury also results in cartilage cell death; hence it impairs the ability of chondrocytes to synthesize macromolecules necessary for repair of the cartilage tissues.

All mechanical induced alterations at the level of the cartilage trigger biochemical processes that ultimately should restore pre-injury homeostasis. Micro-fragmented ECM can generate degradation products of collagen or proteoglycan that leads to activation of MMPs which themselves would amplify the degradation processes. Indeed, MMPs might dramatically increase in synovial fluids within hours after injury. Changes in the ECM constitution can induce processes that trigger proliferation and differentiation of cells that re-synthesize degraded ECM constituents. Mechanistically, the molecules described previously as important elements of the chondrocyte proliferation and differentiation are necessarily involved in these processes. Therefore, substances that modulate their expression can be expected to account for beneficial effects at the level of cartilage reconstitution or renewal and repair.

Inducing or enhancing cartilage repair or cartilage regeneration is defined within the framework of this invention as the formation of new cartilage tissue either by the proliferation of chondrocytes and/or increasing ECM production in cartilage and promote healing of cartilage damage in particular in joints of athletes, other people with a active lifestyle and obese subjects.

In the present invention, the induction or enhancement of cartilage repair or cartilage regeneration is measured by the expression of critical molecules that either participate in the cellular renewal or in the re-building of ECM. Thus, the cartilage tissue of a certain type needed for the restoration of injured or damaged tissue is regenerated from existing viable cells. The new cartilage formation is sufficient to at least partially fill the void or structural discontinuity at the defective site but repair does not, however, mean, or otherwise necessitate, a process of complete healing. With regard to the underlying cellular and molecular modes of action, all parameters described above are considered to be important to reflect the processes that restore trauma-induced defects in the cartilage.

Cartilage injuries are difficult to treat and the injured cartilage does not heal as rapidly or effectively as other body tissues, since cartilage cells have a limited capacity for proliferation and new ECM synthesis. Indeed, chondrocytes undergo an intense phase of proliferation during subchondral growth, but they have limited proliferative capacity at later phases. As a consequence, cartilage defects may remain unrepaired or filled with a functionally inadequate extracellular matrix.

Therefore, there is a great need for agents, in particular derived from natural source, that induce and/ or enhance cartilage repair or cartilage regeneration in particular for the treatment or co-treatment of traumatic cartilage injuries.

Surprisingly, it has been found that carnosol or a carnosol derivative is effective in inducing or enhancing the proliferation of chondrocytes, stimulating the proliferation of cartilage cells and increasing their production of extracellular matrix components which as a consequence promotes cartilage repair and regeneration. Thus, carnosol or a carnosol derivative is useful for maintaining cartilage health, inducing or enhancing cartilage repair or cartilage regeneration as well as for treating cartilage lesions in joints of an animal including humans in particular in people with an active life style, sports people and obese subjects.

Thus, an object of the invention is the use of carnosol or a carnosol derivative for inducing or enhancing cartilage repair or cartilage regeneration. '

Another object of the invention is the use of carnosol or a carnosol derivative for (i) enhancing the proliferation of chondrocytes, (ii) stimulating cartilage cell proliferation and/ or (iii) increasing the synthesis of extracellular matrix, in particular to treat cartilage lesions e.g. induced by traumatic cartilage injuries.

Another further object of the invention is the use of carnosol for maintaining and supporting cartilage health, providing cartilage care, healing cartilage lesions and providing cartilage integrity, i.e. for cartilage care.

In particular, the invention relates to the use of carnosol or a carnosol derivative as mentioned above for maintaining and/or improving joint health, prevention of joint stiffness, increasing mobility of joints, promoting joint mobility, contributing to provide supple and/or flexible joints, lubricating joints, contributing to providing joint integrity, supporting joint function, promoting joint health and function, naturally supporting joint health and mobility for active individual, maintaining the active flexibility of joints and lessening joint problems.

Further the invention relates to the use of carnosol or a carnosol derivative as effective micronutrient for cartilage maintenance, cartilage repair and/ or cartilage regeneration.

In all embodiments of the invention, the term cartilage preferably relates to articular cartilage.

The daily oral dosage of carnosol for humans (usually determined for a 70kg person) is at least 0.1mg. Preferably the daily dosage should be in the range of from about 1 mg/day to about 2000 mg/day, more preferably from about 5 mg/day to about 500 mg/day, most preferably from 10 to 100 mg/day.

In another embodiment, the invention relates to the use of carnosol or a carnosol derivative for the treatment or co-treatment of cartilage injuries in joints, in particular of traumatic cartilage injuries, especially in people with an active life style, sports people and obese subjects.

The term carnosol [2H-9,4a-(Epoxymethano)phenanthren-12-one, CAS 5957-80-2] as used herein refers to synthetic carnosol as well as to carnosol obtainable from natural sources.

The term carnosol derivatives' refers to compounds that are formed from carnosol, structurally related compounds of carnosol or a compound that can be imagined to arise from carnosol if one atom is replaced with another atom or group of atoms. Exemplary derivatives of carnosol encompasses the corresponding esters, mono ethers and diethers thereof such as e.g. C₁-C₅ alkyl esters such as the diacetate (Diacetylcarnosol, CAS 8 684-30-6), dipropionate, dibutyrate or C₁-C₅ alkyl ethers such as 12-monomethylether (12-O-Methylcarnosol, CAS 85514-27-8), 11,12-dimethylether (11,12-Di-O-methylcarnosol, CAS 25460-12-2, CAS 20483-06-1), ethylether, diethyl ether, dibutylether, without being limited thereto. In particular suitable are derivatives which after oral consumption release carnosol via metabolic processes such as the diacetate.

In all embodiments of the invention, preferably carnosol is used, in particular *(4aR,9S,10aS')-*carnosol (I) 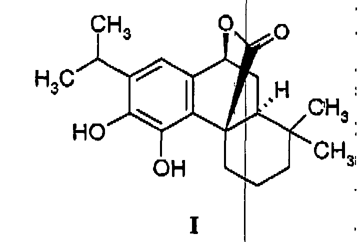 The term "carnosol" also encompasses any material or extract of a plant containing it or them. Preferably, the material or extract of a plant contains carnosol in an amount of at least 30 weight-% (i.e. from 30 to 100 weight-%), preferably in an amount of at least 50 weight-% (i.e. from 50 to 100 weight-%), more preferably in an amount of at least 70 weight-% (i.e. from 70 to 100 weight-%), most preferably in an amount of at least 90 weight-% (i.e, from 90 to 100 weight-%), based on the total weight of the plant material or extract. The terms "material of a plant" and "plant material" used in the context of the present invention mean any part of a plant.

Carnosol can be isolated from plants like the following, but not limited to, *sage, Greek sage and rosemary and rosemary officinalis.* Therefore, any material or extract of these plants or any other plant material or extract containing carnosol in an amount of at least 30 weight-% (i.e. from 30 to 100 weight-%), preferably in an amount of at least 50 weight-% (i.e. from 50 to 100 weight-%), more preferably in an amount of at least 70 weight-% (i.e. from 70 to 100 weight-%), most preferably in an amount of at least 90 weight % (i.e. from 90 to 100 weight-%), based on the total weight of the plant material or extract, is also encompassed by this expression. "Carnosol" means both "natural" (isolated) and "synthetic" (manufactured) carnosol.

Carnosol (CAS: 5957-80-2) is commercially available e.g. at Cayman Chemical Company 1180 East Ellsworth Road, Ann Arbor, Michigan 48108, USA (Catalog Nr. 89800) or at A.G. Scientific, Inc., 6450 Lusk Blvd. Suite E102, San Diego, CA 92121. Furthermore, Carnosol may be synthesized according to methods known in the art as well as be extracted from plant material such as e.g. *sage, greek sage, rosemary and rosemary officinalis.* Optionally, the carnosol content in the plant derived extract may be further enriched e.. by recrystallisation (e.g, from acetic acid) and/ or chromatographic methods known to a skilled person in the art. Furthermore, Carnosol can be formed by conversion of carnosic acid in the presence of a catalyst e.g. via
a) exposing carnosic acid to a solvent comprising a catalyst selected from the group consisting of minor amounts of water, iron, an iron salt, rosemary needles and mixtures thereof, and b) introducing oxygen to produce carnosol. The starting material for this reaction may be any source of carnosic acid. It may be purified carnosic acid itself, or carnosic acid which is not in a purified form, such as a rosemary extract or other plant extract which contains carnosic acid (such as sage, *Salvia spp.),* or it may be rosemary leaves (needles). An exemplary process is illustrated in the examples.

Beside the (pure) compound carnosol especially preferred are plant materials and plant extracts containing at least 30 weight-% (i.e. from 30 to 100- weight-%), preferably at least 50 weight-% (i.e. from 50 to 100 weight-%), more preferably at least 70 weight-% (i.e. from 70 to 100 weight-%), most preferably at least 90 weight-% (i.e. from 90 to 100 weight-%), of carnosol, based on the total weight of the plant material/extract.

According to the present invention not only carnosol itself but also plant materials and extracts containing them in an amount of at least 30 weight-% (i.e. from 30 to 100 weight-%), preferably in an amount of at least 50 weight-% (i.e. from 50 to 100 weight-%), more preferably in an amount of at least 70 weight-% (i.e. from 70 to 100 eight-%), most preferably in an amount of at least 90 weight-% (i.e. from 90 to 100 weight-%), based on the weight of the plant material or extract, as well as dietary and pharmaceutical compositions containing them can be used as medicament, especially for inducing or enhancing the proliferation of chondrocytes, stimulating the proliferation of cartilage cells and increasing their production of extracellular matrix components which, as a consequence, promotes cartilage repair and regeneration.

Preferably the carnosol used is obtained from a natural source e.g. in the form of crystalline carnosol or a carnosol containing extract. In particular carnosol or a carnosol containing extract is derived from rosemary needles.

The dosage is adjusted based on the carnosol content and can easily be determined by a person skilled in the art.

The carnosol derivatives can easily be prepared according to processes known to a person skilled in the art e.g. by methylating or acetylating the phenolic groups, by esterifying carnosol with acetic anhydride or by extraction from natural sources e.g. carnosol-12-methylether from rosemary.

In yet another embodiment, the invention relates to the use of carnosol or a carnosol derivative for the manufacture of a composition for
(a) inducing or enhancing cartilage repair or cartilage regeneration in joints of an animal including humans,
(b) enhancing the proliferation of chondrocytes in joints of an animal including humans,
(c) stimulating cartilage cell proliferation in joints of an animal including humans,
(d) increasing the synthesis of extracellular matrix in joints of an animal including humans and/or
(e) maintaining and supporting cartilage health, providing cartilage care, healing cartilage lesions and providing cartilage integrity, in particular maintaining and/or improving joint health, prevention of joint stiffness, increasing mobility of joints, promoting joint mobility, contributing to provide supple and/or flexible joints, lubricating joints, contributing to providing joint integrity, supporting joint function, promoting joint health and function, naturally supporting joint health and mobility for active individual, maintaining the active flexibility of joints and lessening, joint problems.

In yet a further embodiment, the invention relates to the use of carnosol or a carnosol derivative for the manufacture of a composition for the treatment or co-treatment of cartilage injuries in joints, in particular of traumatic cartilage injuries.

Furthermore, the invention relates to a composition comprising an amount of carnosol or a carnosol derivative which is effective for the treatment or co-treatment of cartilage injuries in joints, in particular of traumatic cartilage injuries in joints of animals including humans.

The compositions according to the invention are especially attractive, since patients have a special interest in treatment considered as "natural" with mild effects and without major side effects, which can be used for disease prevention and as adjuvant treatment.

The compositions according to the invention are in particular useful for inducing or enhancing cartilage repair or cartilage regeneration in people with an active life style, sports people and obese subjects to promote the healing of cartilage and fill the cartilage defects.

In all embodiments of the invention, preferably the compositions are nutraceutical or pharmaceutical, in particular nutraceutical compositions.

In the framework of the invention, with animals is meant all animals, including mammals, examples of which include humans. Preferred examples of mammals beside humans are non-ruminant or ruminant animals including cats, dogs, dromedaries, camels, elephants, and horses. Particular preferred according to the invention are humans, especially people with an active life style, sports people and obese subjects.

The term nutraceutical composition as used herein include food product, foodstuff, dietary supplement, nutritional supplement or a supplement composition for a food product or a foodstuff, preferably beverages (e.g. but not limited to sports beverages, functional waters, juices, smoothies; instant drinks), soups, dairy products (e g. but not limited to single shot yogurt drinks), nutritional bars, and spreads, in particular beverages and nutritional bars.

As used herein, the term food product refers to any food or feed suitable for consumption by humans or animals. The food product may be a prepared and packaged food (e.g., mayonnaise, salad dressing, bread, or cheese food) or an animal feed (*e.g*., extruded and pelleted animal feed, coarse mixed feed or pet food composition). As used herein, the term feedstuff refers to any substance fit for human or animal consumption. The term dietary supplement refers to a small amount of a compound for supplementation of a human or animal diet packaged in single or multiple dose units. Dietary supplements do not generally provide significant amounts of calories but may contain other micronutrients (*e.g*., vitamins or minerals). The term nutritional supplement refers to a composition comprising a dietary supplement in combination with a source of calories. In some embodiments, nutritional supplements are meal replacements or supplements (*e.g*., nutrient or energy bars or nutrient beverages or concentrates).

Food products or foodstuffs are for example beverages such as non-alcoholic and alcoholic drinks as well as liquid preparation to be added to drinking water and liquid food, non-alcoholic drinks are for instance soft drinks, sport drinks, fruit juices, such as for example orange juice, apple juice and grapefruit juice; lemonades, teas, near-water drinks and milk and other dairy drinks such as for example yoghurt drinks, and diet drinks. In another embodiment food products or foodstuffs refer to solid or semi-solid foods comprising the composition according to the invention. These forms can include, but are not limited to baked goods such as cakes and cookies, puddings, dairy products, confections, snack foods, or frozen confections or novelties (*e.g*., ice cream, milk shakes), prepared frozen means, candy, snack products (*e.g*., chips), liquid food such as soups, spreads, sauces, salad dressings, prepared meat products, cheese, yogurt and any other fat or oil containing foods, and food ingredients (*e.g*., wheat flour).
The term food products or foodstuffs also includes functional foods and prepared food products, the latter referring to any pre-packaged food approved for human consumption.

Animal feed including pet food compositions advantageously include food intended to supply necessary dietary requirements, as well as treats (*e.g*, dog biscuits) or other food supplements. The animal feed comprising the composition according to the invention may be in the form of a dry composition (for example, kibble), semi-moist composition, wet composition, or any mixture thereof. Alternatively or additionally, the animal feed is a supplement, such as a gravy, drinking water, yogurt, powder, suspension, chew, treat (*e.g*., biscuits) or any other delivery form.

Dietary supplements of the present invention may be delivered in any suitable format. In preferred embodiments, dietary supplements are formulated for oral delivery. The ingredients of the dietary supplement of this invention are contained in acceptable excipients and/or carriers for oral-consumption. The actual form of the carrier, an thus, the dietary supplement itself, is not critical. The carrier may be a liquid, gel, gelcap, capsule, powder, solid tablet (coated or non-coated), tea, or the like. The dietary supplement is preferably in the form of a tablet or capsule and most preferably in the form of a hard (shell) gelatin capsule. Suitable excipient and/or carriers include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof). Preferred carriers include calcium carbonate, magnesium stearate, maltodextrin, and mixtures thereof. The various ingredients and the excipient and/or carrier are mixed and formed into the desired form using conventional techniques. The tablet or capsule of the present invention may be coated with an enteric coating that dissolves at a pH of about 6.0 to 7.0. A suitable enteric coating that dissolves in the small intestine but not in the stomach is cellulose acetate phthalate. Further details on techniques for formulation for and administration may be found in the latest edition of Remington's Pharmaceutical Sciences (Maack Publishing Co., Easton, PA).

In other embodiments, the dietary supplement is provided as a powder or liquid suitable for adding by the consumer to a food or beverage. For example, in some embodiments, the dietary supplement can be administered to an individual in the form of a powder, for instance to be used by mixing into a beverage, or by stirring into a semi-solid food such as a pudding, topping, sauce, puree, cooked cereal, or salad dressing, for instance, or by otherwise adding to a food e.g. enclosed in caps of food or beverage container for release immediately before consumption. The dietary supplement may comprise one or more inert ingredients, especially if it is desirable to limit the number of calories added to the diet by the dietary supplement. For example, the dietary supplement of the present invention may also contain optional ingredients including, for example, herbs, vitamins, minerals, enhancers, colorants, sweeteners, flavorants, inert ingredients, and the like.
In some embodiments, the dietary supplements further comprise vitamins and minerals including, but not limited to, calcium phosphate or acetate tribasic; potassium phosphate, dibasic; magnesium sulfate or oxide; salt (sodium chloride); potassium chloride or acetate; ascorbic acid; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D3; cyanocobalamin; sodium selenite; copper sulfate; vitamin A; vitamin C; inositol; potassium iodide. Suitable dosages for vitamins and minerals may be obtained, for example, by consulting the U.S.RDA guidelines.

In other embodiments, the present invention provides nutritional supplements (*e.g*., energy bars or meal replacement bars or beverages) comprising the composition according to the invention. The nutritional supplement may serve as meal or snack replacement and generally provide nutrient calories. Preferably, the nutritional supplements provide carbohydrates, proteins, and fats in balanced amounts. The nutritional supplement can further comprise carbohydrate, simple, medium chain length, or polysaccharides, or a combination thereof. A simple sugar can be chosen for desirable organoleptic properties. Uncooked cornstarch is one example of a complex carbohydrate. If it is desired that it should maintain its high molecular weight structure, it should be included only in food formulations or portions thereof which are not cooked or heat processed since the heat will break down the complex carbohydrate into simple carbohydrates, wherein simple carbohydrates are mono- or disaccharides. The nutritional supplement contains, in one embodiment, combinations of sources of carbohydrate of three levels of chain length (simple, medium and complex; e.g., sucrose, maltodextrins, and uncooked cornstarch).
Sources of protein to be incorporated into the nutritional supplement of the invention can be any suitable protein utilized in nutritional formulations and can include whey protein, whey protein concentrate, whey powder, egg, soy flour, soy milk, soy protein, soy protein isolate, caseinate (e.g., sodium caseinate, sodium calcium caseinate, calcium caseinate, potassium caseinate), animal and vegetable protein and hydrolysates or mixtures thereof. When choosing a protein source, the biological value of the protein should be considered first, with the highest biological values being found in caseinate, whey, lactalbumin, egg albumin and whole egg proteins. In a preferred embodiment, the protein is a combination of whey protein concentrate and calcium caseinate. These proteins have high biological value; that is, they have a high proportion of the essential amino acids. See Modem Nutrition in Health and Disease, eighth edition, Lea & Febiger, publishers, 1986, especially Volume 1, pages 30-32.
The nutritional supplement can also contain other ingredients, such as one or a combination of other vitamins, minerals, antioxidants, fiber and other dietary supplements (*e.g*., protein, amino acids, choline, lecithin, omega-3 fatty acids). Selection of one or several of these ingredients is a matter of formulation, design, consumer preference and end-user. The amounts of these ingredients added to the dietary supplements of this invention are readily known to the skilled artisan. Guidance to such amounts can be provided by the U.S. RDA doses for children and adults. Further vitamins and minerals that can be added include, but are not limited to, calcium phosphate or acetate, tribasic; potassium phosphate, dibasic; magnesium sulfate or oxide; salt (sodium chloride); potassium chloride or acetate ascorbic acid; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D3; cyanocobalamin; sodium selenite; copper sulfate; vitamin A; vitamin C; Vitamin E, inositol; potassium iodide,

The nutritional supplement can be provided in a variety of forms, and by a variety of production methods. In a preferred embodiment, to manufacture a food bar, the liquid ingredients are cooked; the dry ingredients are added with the liquid ingredients in a mixer and mixed until the dough phase is reached; the dough is put into an extruder, and extruded; the extruded dough is cut into appropriate lengths; and the product is cooled. The bars may contain other nutrients and fillers to enhance taste, in addition to the ingredients specifically listed herein.
It is understood by those of skill in the art that other ingredients can be added to those described herein, for example, fillers, emulsifiers, preservatives, etc. for the processing or manufacture of a nutritional supplement.

Additionally, flavors, coloring agents, spices, nuts and the like may be incorporated into the nutraceutical composition. Flavorings can be in the form of flavored extracts, volatile oils, chocolate flavorings, peanut butter flavoring, cookie crumbs, crisp rice, vanilla or any commercially available flavoring. Examples of useful flavoring include, but are not limited to, pure anise extract, imitation banana extract, imitation cherry extract, chocolate extract, pure lemon extract, pure orange extract, pure peppermint extract, imitation pineapple extract, imitation rum extract, imitation strawberry extract, or pure vanilla extract; or volatile oils, such as balm oil, bay oil, bergamot oil, cedarwood oil, walnut oil, cherry oil, cinnamon oil, clove oil, or peppermint oil; peanut butter, chocolate flavoring, vanilla cookie crumb, butterscotch or toffee. In one embodiment, the dietary supplement contains cocoa or chocolate.

Emulsifiers may be added for stability of the nutraceutical compositions. Examples of suitable emulsifiers include, but are not limited to, lecithin (*e.g*., from egg or soy), and/or mono- and diglycerides. Other emulsifiers are readily apparent to the skilled artisan and selection of suitable emulsifier(s) will depend, in part, upon the formulation and final product. Preservatives may also be added to the nutritional supplement to extend product shelf life. Preferably, preservatives such as potassium sorbate, sodium sorbate, potassium benzoate, sodium benzoate or calcium disodium EDTA are used.
In addition to the carbohydrates described above, the nutraceutical composition can contain natural or artificial (preferably low calorie) sweeteners, *e.g*., saccharides, cyclamates, aspartamine, aspartame, acesulfame K, and/or sorbitol. Such artificial sweeteners can be desirable if the nutritional supplement is intended to be consumed by an overweight or obese individual, or an individual with type II diabetes who is prone to hyperglycemia.
Moreover, a multi-vitamin and mineral supplement may be added to the nutraceutical compositions of the present invention to obtain an adequate amount of an essential nutrient, which is missing in some diets. The multi-vitamin and mineral supplement may also be useful for disease prevention and protection against nutritional losses and deficiencies due to lifestyle patterns.

The dosage and ratios of carnosol or a carnosol derivative administered via a nutraceutical composition will, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired which can be determined by the expert in the field with normal trials, or with the usual considerations regarding the formulation of a nutraceutical composition.

A food or beverage suitably contains about 0.5 mg to about 1000 mg of carnosol or a carnosol derivative per serving. If the composition is a pharmaceutical composition, such a composition may contain carnosol or a carnosol derivative in an amount from about 1 mg to about 2000 mg per dosage unit, e.g., per capsule or tablet, or from about 1 mg per daily dose to about 3000 mg per daily dose of a liquid formulation.

The pharmaceutical compositions according to the invention preferably further comprise pharmaceutically acceptable carriers. Suitable pharmaceutical carriers are *e.g*. described in Remington's Pharmaceutical Sciences, (vide supra), a standard reference text in this field. Examples of such pharmaceutically acceptable carriers are both inorganic and organic carrier materials, suitable for oral/parenteral/injectable administration and include water, gelatin, gum arabic, lactose, starch, magnesium stearate, talc, vegetable oils, and the like.

The pharmaceutical composition may further comprise conventional pharmaceutical additives and adjuvants, excipients or diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffer, lubricants, colorants, wetting agents, fillers, and the like.

The dosages and ratios of the individual components in a pharmaceutical composition can be determined by the expert in the field with normal preclinical and clinical trials, or with the usual considerations regarding the formulation of pharmaceutical composition.

In a preferred embodiment carnosol or a carnosol derivative is administered via a pharmaceutical composition either in the form of a single dose or by multiple doses in an amount of at least 0-1 mg/ kg bodyweight/ day, preferably in an amount of 0.1-450 mg/ kg body weight/ day, most preferably in an amount of 0.1-140 mg/ kg body weight / day.

The compositions according to the present invention may be in any galenic form that is suitable for administering to the animal body including the human body, more in particular in any form that is conventional for oral administration, e.g. in solid form, for example as (additives/supplements for) food or feed, food or feed premixes, fortified food or feed, tablets, pills, granules, dragées, capsules, and effervescent formulations such as powders and tablets, or in liquid form, for instance in the form of solutions, emulsions or suspensions, for example as beverages, pastes and oily suspensions. The pastes may be filled into hard or soft shell capsules, whereby the capsules feature e.g. a matrix of (fish, swine, poultry, cow) gelatin, plant proteins or ligninsulfonate. Examples for other application forms are forms for transdermal, parenteral, topical or injectable administration. The nutraceutical and pharmaceutical compositions may be in the form of controlled (delayed) release formulations. Examples of pharmaceutical compositions also include compositions suitable for topical application and transdermal absorption of the phenolic compound, such as crèmes, gels, sprays, dry sticks, powders etc.
In a preferred embodiment the compositions according to the invention are in the form of a tablet, a pill, a granule, a dragée, a capsule or an effervescent formulation.

The compositions according to the invention may also contain further active ingredients suitable for joint care e.g. selected from antioxidants, including vitamins C and E, chondroitin sulphate, hydrolyzed collagen (hydrolysate), ginger (rhizome) extract glucosamine, methylsulfonylmethane (MSM), S-adenosyl methionine, selenium, vitamins B9 (folate), polyunsaturated fatty acids (PUFA's), B12 (cobalamin) and Vitamin D3, preferably selected from Vitamin E, PUFA's, chondroitin sulphate and glucosamine.

Polyunsaturated fatty acids, which are suitable according to the present invention, are mono- or polyunsaturated carboxylic acids having preferably 16 to 24 carbon atoms and, in particular, 1 to 6 double bonds, particularly preferably having 4 or 5 or 6 double bonds.

The unsaturated fatty acids can belong both to the n-6 series and to the n-3 series. Polyunsaturated fatty acids of the n-3 series are preferred. Preferred examples of n-3 polyunsaturated acids are eicosapenta-5,8,11,14,17-enoic acid (EPA) and docosahexa-4,7,10,13,16,19-enoic acid (DHA), as well as arachidonic acid (ARA).

Preferred derivatives of the polyunsaturated fatty acids are their esters, for example glycerides and, in particular, triglycerides; particularly preferably the ethyl esters. Triglycerides of n-3 polyunsaturated fatty acids are especially preferred.

The triglycerides can contain 3 uniform unsaturated fatty acids or 2 or 3 different unsaturated fatty acids. They may also partly contain saturated fatty acids.

When the derivatives are triglycerides, normally three different n-3 polyunsaturated fatty acids are esterified with glycerin. In one preferred embodiment of the present invention triglycerides are used, whereby 30 % of the fatty acid part are n-3 fatty acids and of these 25 % are long-chain polyunsaturated fatty acids. In a further preferred embodiment commercially available ROPUFA® '30' n-3 Food Oil (DSM Nutritional Products Ltd, Kaiseraugst, Switzerland) is used. In another preferred embodiment of the present invention the PUFA ester is ROPUFA^{®} '75' n-3 EE. ROPUFA '75' n-3 EE is refined marine oil in form of an ethyl ester with minimum content of 72 % n-3 fatty acid ethyl ester. It is stabilized with mixed tocopherols, ascorbyl palmitate, citric acid and contains rosemary extract.

According to the present invention it can be advantageous to use naturally occuring oils (one ore more components) containing triglycerides of polyunsaturated fatty acids, for example plant oils.

Preferred oils which comprise triglycerides of polyunsaturated fatty acids are olive oil, sunflower seed oil, evening primrose seed oil, borage oil, grape seed oil, soybean oil, groundnut oil, wheat germ oil, pumpkin seed oil, walnut oil, sesame seed oil, rapeseed oil (canola), blackcurrant seed oil, kiwifruit seed oil, oil from specific fungi and fish oils.

Alternatively other polyunsaturated fatty acids (e.g. omega-3 fatty acids; omega-6 fatty acids) and/or their derivatives may be used.

The term Vitamin E encompasses tocopherol, tocopherol derivatives (such as vitamin-E-acetate) as well as mixtures of nat. vitamin E.

Furthermore, the invention relates to the use of a composition according to the invention for maintaining and supporting cartilage health, providing cartilage care, healing cartilage lesions and providing cartilage integrity, i.e. for cartilage care in particular for maintaining and/or improving joint health, prevention of joint stiffness, increasing mobility of joints, promoting joint mobility, providing supple and/or flexible joint, contributing to provide supple and/or flexible joints, lubricating joints, contributing to providing joint integrity, supporting joint function, promoting joint health and function, naturally supporting joint health and mobility for active individual, maintaining the active flexibility of joints and lessening joint problems.

Further objects of the present invention are the following methods:
- A method for the treatment or co-treatment of cartilage injuries, in particular traumatic cartilage injuries in animals including humans said method comprising the step of administering an effective amount of carnosol or a carnosol derivative to animals including humans, which are in need thereof.
- A method of cartilage repair or regeneration in animals including humans said method comprising the step of administering an effective amount of carnosol or a carnosol derivative to animals including humans, which are in need thereof.
- A method of (a) inducing or enhancing cartilage repair or cartilage regeneration in joints of an animal including humans, (b) enhancing the proliferation of chondrocytes, (c) stimulating cartilage cell proliferation, (c) stimulating cartilage cell proliferation in joints of an animal including humans, (d) increasing the synthesis of extracellular matrix in joints of an animal including humans and/ or (e) maintaining and supporting cartilage health, providing cartilage care, healing cartilage lesions and providing cartilage integrity, in particular for maintaining and/or improving joint health, prevention of joint stiffness, increasing mobility of joints, promoting joint mobility, providing supple and/or flexible joint, contributing to provide supple and/or flexible joints, lubricating joints, contributing to providing joint integrity, supporting joint function, promoting joint health and function, naturally supporting joint health and mobility for active individual, maintaining the active flexibility of jointsand lessening joint problems, said method comprising the step of administering an effective amount of carnosol or a carnosol derivative to animals including humans, which are in need thereof.

Effective amount of carnosol or a carnosol derivative in these methods refers to an amount necessary to obtain a physiological effect. The physiological effect may be achieved by one single dose or by repeated doses. The dosage administered may, of course, vary depending upon known factors, such as the physiological characteristics of the particular composition and its mode and route of administration; the age, health and weight of the recipient, the nature and extent of the symptoms; the kind of concurrent treatment; the frequency of treatment; and the effect desired and can be adjusted by a person skilled in the art.
Preferably, the effective amount of carnosol or a carnosol derivative in the methods above is such that the concentration of carnosol or a carnosol derivative in the blood plasma after administration ranges between 1 and 20 µM.

In a preferred embodiment of these methods, carnosol or a carnosol derivative is combined with at least one compound selected from antioxidants, including vitamins C and E, chondroitin sulphate, PUFA's, hydrolyzed collagen (hydrolysate), ginger (rhizome) extract, glucosamine, methylsulfonylmethane (MSM), S-adenosyl methionine, selenium, vitamin B9 (folate), B12 (cobalamin), and Vitamin D3 in particular selected from Vitamin E, glucosamine, chondroitin sulphate and PUFA's.

The invention will now be elucidated by way of the following examples, without however being limited thereto.

### Examples

### Example 1 Preparation of Carnosol

102g rosemary extract '"Naturex CS" was stirred in 320ml ethylacetate and filtered to remove the insolubles. The filtrate was evaporated to dryness to obtain 34g residue. To 34g of the residue was added 280ml acetic acid and 5.7g sodium hydrogen carbonate, and 15mg iron chloride hexahydrate in 50ml of water. This was added to a 500 ml reaction vessel. The mixture was stirred at ambient temperature in an atmosphere of oxygen for 4d. The slurry of carnosol was filtrated and dried in the vacuum yielding 12.5g carnosol of purity = 92%.
Repeated recrystallisation from acetic acid yielded carnosol of >= 95% purity

### Example 2: Soft gelatin capsule

Soft gelatin capsules are prepared by conventional procedures providing a dose of carnosol of 100 mg per capsule. A suitable daily dose is 1 to 5 capsules.

Other ingredients: glycerol. Water, gelatine, vegetable oil

### Example 3: Hard gelatin capsule

Hard gelatin capsules are prepared by conventional procedures providing a dose of carnosol of 75 mg per capsule. A suitable daily dose is 1 to 5 capsules.
Other ingredients:
Fillers: lactose or cellulose or cellulose derivatives q.s.
Lubricant: magnesium stearate if necessary (0.5%)

### Example 4: Tablet

Tablets are prepared by conventional procedures providing as active ingredient 100 mg of carnosol per tablet, and as excipients microcrystalline cellulose, silicone dioxide (SiO₂), magnesium stearate, crosscarmellose sodium ad 500 mg.

### Example 5; soft drink

A soft drink containing a phenolic compound may be prepared as follows:
A soft drink is prepared from the following ingredients:

| Ingredient | [g] |
|---|---|
| A. juice concentrates and water soluble flavours | |
| 60.3°Brix, 5.15% acidity | 657.99 |
| 43.5° Brix, 32.7% acidity | 95.96 |
| Orange flavour, water soluble | 3.43 |
| Apricot flavour, water soluble | 6.71 |
| Water | 26.46 |

| B. color | |
|---|---|
| β-carotene 10% CWS | 0.89 |
| Water | 67.65 |

| C. Acid and antioxidant | |
|---|---|
| Ascorbic acid | 4.11 |
| Citric acid anhydrous | 0.69 |
| Water | 43.18 |

| D. stabilizers | |
|---|---|
| Pectin | 0.20 |
| Sodium benzoate | 2.74 |
| Water | 65.60 |

| E. oil soluble flavours | |
|---|---|
| Orange flavour, oil soluble | 0.34 |
| Orange oil distilled | 0.34 |

| F. active ingredient | |
|---|---|
| Carnosol | 50 mg |

Fruit juice concentrates and water soluble flavours are mixed without incorporation of air. The color is dissolved in deionized water. Ascorbic acid and citric acid are dissolved in water. Sodium benzoate is dissolved in water. The pectin is added under stirring and dissolved while boiling. The solution is cooled down. Orange oil and oil soluble flavours are premixed. The active ingredient as mentioned under F is stirred into the fruit juice concentrate mixture of A.

In order to prepare the soft drinks all components A-F are mixed together before homogenizing using a Turrax and then a high-pressure homogenizer (p₁ = 200 bar, p₂ = 50 bar).

### Example 6

Influence of carnosol on the expression of genes involved in the build-up and break-down of cartilage.
Carnosol of >= 95% purity prepared as disclosed in example 1 was used.

The effect of carnosol on the expression of human collagen, aggrecan and MMP genes was measured *in vitro* in normal human chondrocytes (derived from knee). Control experiments without carnosol were done concomitantly to compare the expression of these genes or proteins to that in un-stimulated cells.
The effect of carnosol (at 1 µmol/L) on the expression of anabolic genes (see Table B) was measured in normal human chondrocytes obtained from the knee of healthy donors (HNAC-kn, Cambrex, #CC2250). These primary chondrocytes were passaged *in vitro* not more than 4 times, before being used for experiments. Cells were cultured in differentiation medium (Cambrex, #CC.3225) for 5 - 10 days. Total RNA was extracted from cultured cells and reverse-transcribed; expression levels of distinct genes were determined by quantitative real-time PCR as detailed by Richard et al. Mol. Nutr. Food Res. 49, 431-442, (2005).

Effect of carnosol on anabolic gene expression in normal human primary chondrocytes
Numbers indicate the level of gene expression (in % of control-treated cells)

| *type of gene* | *gene* | *5 days culture* | *10 days culture* | | *effect of expression* | *carnosol on gene* | | |
|---|---|---|---|---|---|---|---|---|
| Anabolic | Aggrecan | 139 | 97 | | Increased | | | |
| Anabolic | Collagen I | 207 | 98 | | Increased | | | |
| Anabolic | Collagen II | 128 | 189 | | Increased | | | |
| Anabolic | SOX-5 | 85 | 126 | | Increased | | | |
| Anabolic | SOX-9 | 112 | 93 | | Increased | | | |

Anabolic gene expression (*i.e*. favoring cartilage rebuilding), including aggrecan and collagen II, substantially increased in primary chondrocytes that were cultured in the presence of carnosol. Similarly, the expression of two transcription factors (SOX-5 and SOX-9), that favor the proliferation of ECM-rebuilding chondrocytes and therefore also the expression of collagen was enhanced. Thus, carnosol is surprisingly effective in inducing or enhancing cartilage repair or cartilage regeneration by stimulating the proliferation of cartilage cells and increasing the production of extracellular matrix (ECM) components to regenerate cartilage and offset the cartilage defects.

The increased build-up of the ECM has been directly shown by measuring the production of collagen II. To this aim, chondrocytes were cultured in differentiation medium containing 20 µmol/L carnosol for 10 days. Cellular collagen II was then quantified by enzyme linked immunosorbent assay (ELISA). As shown in Table 2, carnosol significantly increased the collagen production in chondrocytes.

**Table 2**

| *Treatment(10 days of culture)* | *% of collagen* | *Effect of carnosol on protein expression* | |
|---|---|---|---|
| vehicle | 100 | - (control) | |
| Carnosol (20 µmol/L) | **129** | **Increased** | |

The data shows that carnosol exerts multiple effects on chondrocytes that result in improved cartilage build-up or regeneration as well as in diminished cartilage destruction.

### Example 7:

A 39 year former amateur football player started to play again without training. In the amateur game while dribbling round a defender he is pushed by another player and twisted his knee. He experienced knee swelling and pain. The medical examination and x-ray reveals severe traumatic meniscus tear and damage to other part of the knee. Magnetic resonance imaging confirms the diagnosis and shows also articular cartilage damage. After surgical repair and standard therapy he takes 200 mg of carnosol per day to support the repair and healing of the damaged cartilage.

### Example 8

A 35 year recreational skier fell after a jump and twisted his leg. In the night the keen swell and was painful. A physician examines the knee and rulers out other possible causes of knee pain unrelated to the injury by x-ray and magnetic resonance imaging. Magnetic resonance imaging also reveals cartilage damage. The knee injury is treated with standard therapy (rest, ice, compression and elevation). In addition he takes 100 mg of carnosol per day to support a high rate of cartilage repair and healing.

## Claims

1. Use of carnosol or a carnosol derivative for inducing or enhancing cartilage repair or cartilage regeneration.

2. Use of carnosol or a carnosol derivative for inducing or enhancing the proliferation of chondrocytes.

3. Use of carnosol or a carnosol derivative for stimulating cartilage cell proliferation.

4. Use of carnosol or a carnosol derivative for increasing the synthesis of extracellular matrix.

5. Use of carnosol or a carnosol derivative for maintaining and supporting cartilage health, providing cartilage care, healing cartilage lesions and providing cartilage integrity.

6. Use of carnosol or a carnosol derivative according to any one of claims 1 to 5 for maintaining and/or improving joint health, prevention of joint stiffness, increasing mobility of joints, promoting joint mobility, contributing to provide supple and/or flexible joints, lubricating joints, contributing to providing joint integrity, supporting joint function, promoting joint health and function, naturally supporting joint health and mobility for active individual, maintaining the active flexibility of joints and lessening joint problems.

7. Use of carnosol or a carnosol derivative as effective micronutrient for cartilage maintenance, cartilage repair and/ or cartilage regeneration.

8. Use of carnosol or a carnosol derivative for the treatment or co-treatment of cartilage injuries in joints, in particular of traumatic cartilage injuries especially in people with an active life style, sports people and obese subjects.

9. Use of carnosol or a carnosol derivative for the manufacture of a composition for (a) inducing or enhancing cartilage repair or cartilage regeneration, (b) enhancing the proliferation of chondrocytes, (c) stimulating cartilage cell proliferation, (d) increasing the synthesis of extracellular matrix, (e) maintaining and supporting cartilage health, providing cartilage care, healing cartilage lesions and providing cartilage integrity, in particular for maintaining and/or improving joint health, prevention of joint stiffness, increasing mobility of joints, promoting joint mobility, contributing to provide supple and/or flexible joint, lubricating joints, contributing to providing joint integrity, supporting joint function, promoting joint health and function, naturally supporting joint health and mobility for active individual, maintaining the active flexibility of joints and lessening joint problems.

10. Use of carnosol or a carnosol derivative for the manufacture of a composition for the treatment or co-treatment of cartilage injuries in joints.

11. Use according to claim 10, wherein the cartilage injuries are traumatic cartilage injuries.

12. Use according to any one of claims 9 to 11, wherein the composition is a nutraceutical composition.

13. The use according to any one of claims 9 - 12, wherein the composition is a beverage, soup, dairy product, nutritional bar or spread, in particular a beverage or a nutritional bar.

14. The use according to any one of claims 9 - 12, wherein the composition is in the form of a tablet, a pill, a granule, a dragée, a capsule or an effervescent formulation.

15. A method for the treatment or co-treatment of cartilage injuries, in particular traumatic cartilage injuries in animals including humans said method comprising the step of administering an effective amount of carnosol or a carnosol derivative to animals including humans, which are in need thereof.

16. A method of cartilage repair or regeneration in animals including humans said method comprising the step of administering an effective amount of carnosol or carnosol derivative to animals including humans, which are in need thereof.

17. A method of (a) inducing or enhancing cartilage repair or cartilage regeneration in joints of an animal including humans, (b) enhancing the proliferation of chondrocytes, (c) stimulating cartilage cell proliferation, (d) increasing the synthesis of extracellular matrix in joints and/ or (e) maintaining and supporting cartilage health, providing cartilage care, healing cartilage lesions and providing cartilage integrity, in particular for maintaining and/or improving joint health, prevention of joint stiffness, increasing mobility of joints, promoting joint mobility, contributing to provide supple and/or flexible joints, lubricating joint, contributing to providing joint integrity, supporting joint function, promoting joint health and function, naturally supporting joint health and mobility for active individual, maintaining the active flexibility of joints and lessening joint problems, said method comprising the step of administering an effective amount of carnosol or a carnosol derivative to animals including humans, which are in need thereof.

18. Use of a composition according to claim 18 for maintaining and supporting cartilage health, providing cartilage care, healing cartilage lesions and providing cartilage integrity in particular for maintaining and/or improving joint health, prevention of joint stiffness, increasing mobility of joints, promoting joint mobility, contributing to provide supple and/or flexible joints, lubricating joint, contributing to providing joint integrity, supporting joint function, promoting joint health and function, naturally supporting joint health and mobility for active individual, maintaining the active flexibility of joints and lessening joint problems.
